# EUROPÄISCHE PATENTANMELDUNG

(11) **EP 1 010 756 A1**
(43) Veröffentlichungstag der Anmeldung: **21.06.2000**
(21) Anmeldenummer: 99121099.8
(22) Anmeldetag: 22.10.1999
(51) Int. Cl.: C12N 5/06

(54) **Mesothelzellen der viszeralen Pleura**

(30) Priorität: 18.12.1998 DE 19858549
(71) Anmelder: Bergbau-Berufsgenossenschaft, 44789 Bochum (DE)
(72) Erfinder: Voss, Bruno, Dr., 48301 Nottuln (DE); Böhm, Sabine, 44799 Bochum (DE)
(74) Vertreter: Cohausz & Florack

(57) **Zusammenfassung**

In einem Verfahren zur Bereitstellung von Mesothelzellen oder einer Mesothelzellinie aus der viszeralen Pleura von Säugern wird eine isolierte Säugerlunge in einem ein Proteasegemisch enthaltenden, serumfreien Inkubationspuffer unter Bewegung bei etwa 37°C inkubiert, die ausgelösten Zellen isoliert und mit einem serumfreien Kulturmedium aufnimmt, wobei das Proteasegemisch vorzugsweise ein Gemisch aus Kollagenase Typ IV und Dispase ist.

## Beschreibung

Die Erfindung betrifft die Verfahren zur Bereitstellung von Primärzellen aus dem Mesothel der viszeralen Pleura sowie eine daraus erhaltene Zellinie.

Die Pleura visceralis (Pleura pulmonalis) ist die Hülle, die jeden Lungenflügel eines Säugers überzieht. Die Pleura visceralis grenzt, nur durch einen engen Flüssigkeitsspalt getrennt, an die Pleura parietalis, die die innere Thoraxwand, das Zwerchfell und das Mediastinum seitlich überzieht. Das Mesothel der Pleura visceralis ist ein einschichtiges, niedrigprismatisches Plattenepithel.

Das Mesotheliom, ein Tumor des Mesothels der Pleura, wird in zwei von drei Fällen durch Asbest hervorgerufen. Aber auch andere, den Menschen am Arbeitsplatz gefährdende Stoffe können ein Mesotheliom verursachen. Die Überprüfung der toxischen Wirkung von Stoffen auf die Pleura visceralis war bisher nur an vollständigen Geweben möglich, weil Zellen der Pleura visceralis nicht ohne weitere Zellen oder Bestandteile der Pleura isoliert werden konnten. Mesothelzellen der viszeralen Pleura in Reinkultur waren bisher nicht erhältlich.

Die Gewinnung solcher Zellen brächte erhebliche Vorteile für sogenannte Zytotoxizitätstests und zur Gewinnung von Collagen Typ I. In standardisierten Tests könnte die Wirkung von Stoffen auf die Pleura visceralis schnell überprüft werden, ohne daß wie bisher beispielsweise eine Rattenlunge isoliert werden müßte. Ferner wird Collagen Typ I in den Mesothelzellen der Pleura visceralis in vergleichbarem Ausmaß gebildet wie ansonsten nur in den Fibroblasten. Folglich erlauben diese Zellen auch biochemische Untersuchungen zur Collagensynthese.

Der Erfindung lag also die Aufgabe zugrunde, Mesothelzellen für eine Vielzahl von biochemischen Untersuchungen und zur Gewinnung von Collagen Typ I bereitzustellen. Dies gilt sowohl für Primärzellen als auch aus den Primärzellen entwickelte Zellinien.

Gelöst wird diese Aufgabe durch ein Verfahren zur Bereitstellung von Zellen oder einer Zellinie aus der viszeralen Pleura von Säugern, wobei man eine isolierte Säugerlunge in einem ein Proteasegemisch enthaltenden, serumfreien Inkubationspuffer unter Bewegung bei etwa 37°C inkubiert, die ausgelösten Zellen isoliert und mit einem serumfreien Kulturmedium aufnimmt.

Es war überraschend, daß eine Hauptvoraussetzung zur erfolgreichen Isolierung und Züchtung von Mesothelzellen der viszeralen Pleura die Serumfreiheit des Inkubationspuffers oder Züchtungspuffers ist. Mit Ausnahme von Tumorzellen und Hautfibroblasten werden Zellen üblicherweise mit Serum gezogen. Im Fall der Benutzung eines serumfreien Kulturmediums war zu erwarten, daß nur eine ganz geringe Anzahl von Passagen möglich ist. Es war ferner zu erwarten, daß einerseits kein vernünftiges Wachstum in einem serumfreien Kulturmedium möglich ist und biochemische Untersuchungen nur über einen geringen Zeitraum wegen der zu erwartenden Degeneration der Mesothelzellen der viszeralen Pleura in einem serumfreien Medium möglich sind.

Ein geeigneter Puffer zur Inkubation der Säugerlunge ist beispielsweise ein üblicher PBS-Puffer.

Als Proteasen zur Herauslösung der Mesothelzellen aus der viszeralen Pleura ist ein Gemisch aus Collagenase Typ IV und Dispase besonders bevorzugt. Weitere Proteasen sind einsetzbar wie beispielsweise Elastase, die jedoch eine längere Behandlungsdauer erfordert.

Die Konzentration der Kollagenase Typ IV in dem Inkubationspuffer kann 120 bis 180 U/mg/ml, vorzugsweise etwa 150 U/mg/ml, betragen. Die Konzentration der Dispase in dem Inkubationspuffer kann 1 bis 5 U/mg/ml, vorzugsweise etwa 3 mg/ml, betragen. Die Inkubationsdauer kann beispielsweise 5 bis 40 Minuten, beispielsweise 10 bis 30 Minuten und in am meisten bevorzugter Weise etwa 20 Minuten betragen. D

Nachfolgend wird ein bevorzugtes Verfahren zur Gewinnung von viszeralen Mesothelzellen beschrieben. Männliche Ratten des Stamms 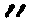 Sprague-Dawley" mit einem Gewicht von etwa 180 g erhielten eine Halothan-Narkose in Überdosis. Nach Freipräparation der Lunge konnte sie über die rechte Lungenarterie entblutet werden, so daß die Lunge ein weißliches Aussehen erhielt. Anschließend wurde die Lunge entnommen. Die Trachea wurde mit einer Arterienklemme verschlossen. Die Lunge wurde zur Isolierung der viszeralen Mesothelzellen in 50 ml eines auf 37°C erwärmten Enzymgemischs aus Collagenase Typ IV (etwa 150 µ/mg/ml PBS) und Dispase (etwa 3 mg/ml PBS) gelegt. Die Inkubation in einem Rotator dauert etwa 30 Minuten bei einer Temperatur von etwa 37°C. Eine Überkopfrotation der Lunge in dem Inkubationspuffer hat sich als besonders wirksam erwiesen. Nach Inkubation wurde die Lunge aus dem Pufferbad entnommen. Das Enzymgemisch mit den gelösten Zellen wurde etwa 10 Minuten bei 250 G und einer Temperatur von etwa 10°C zentrifugiert. Der Überstand wurde verworfen und das zellhaltige Pellet in 5 ml eines Kulturmediums, beispielsweise Panserin® aufgenommen.

Die primären Zellen wurden in Plastik-Zellkulturflaschen (25 cm³ Primaria-Kulturflasche) abgefüllt und nach 24 Stunden erfolgte der erste Wechsel des Kulturmediums. Ein weiterer Wechsel folgte an jedem dritten Tag. Die Zellen wurden bei 37°C, 5 % CO₂ und gesättigter Luftfeuchtigkeit gezüchtet.

Die histologische Begutachtung der Lunge vor und nach der Enzymbehandlung zeigt eine intakte Lungenstruktur mit einer intakten Elastica der Pleura. Bei den isolierten Zellen handelt es sich um einkernige Zellen mit einem doppelten Chromosomensatz, wie er für somatische Zellen üblich ist. In der Zellkultur zeigen die Zellen ein typisches epitheliales Wachstum. Ein typisches weiteres Merkmal ist ihre positive Reaktion mit Anti-Zytokeratin.

Zur Immortalisation der Zellen wurden die Mesothelzellen nach Erreichen der Konfluenz mit einer Trypsin-Lösung (0,25 % Trypsin von Serva) von dem Kulturflaschenboden gelöst und vereinzelt. Die weitere Kultur erfolgte in 75 cm³ Falcon-Kulturflaschen und wurde bis zur 70. Passage durchgeführt. Die eingefrorenen Zellen lassen sich nach dem Auftauen wieder kultivieren und weiter vermehren. Das Einfriermedium besteht aus 98 Gew.% Kulturmedium und 2 Gew.% Dimethylsulfoxid.

## Patentansprüche

1. Verfahren zur Bereitstellung von Zellen oder einer Zellinie aus der viszeralen Pleura von Säugern, **dadurch gekennzeichnet**, daß man eine isolierte Säugerlunge in einem ein Proteasegemisch enthaltenden, serumfreien Inkubationspuffer bei etwa 37°C inkubiert, die ausgelösten Zellen isoliert und mit einem serumfreien Kulturmedium aufnimmt.

2. Verfahren nach Anspruch 1, **dadurch gekennzeichnet**, daß das Proteasegemisch ein Gemisch aus Kollagenase Typ IV und Dispase ist.

3. Verfahren nach Anspruch 2, **dadurch gekennzeichnet**, daß die Konzentration der Kollagenase Typ IV in dem Inkubationspuffer 120 bis 180 U/mg/ml, vorzugsweise etwa 150 U/mg/ml beträgt.

4. Verfahren nach Anspruch 2, **dadurch gekennzeichnet**, daß die Konzentration der Dispase in dem Inkubationspuffer 1 bis 5 U/mg/ml, vorzugsweise etwa 3 mg/ml beträgt.

5. Mesothelzellen der viszeralen Pleura und eine daraus abgeleitete Zellinie, die erhältlich sind durch Inkubation einer isolierten Säugerlunge in einem ein Proteasegemisch enthaltenden, serumfreien Inkubationspuffer bei etwa 37°C über einen Zeitraum bis die gewünschte Zellausbeute erreicht wird, durch Isolation der ausgelösten Zellen und Aufnahme mit einem serumfreien Kulturmedium.
